# EUROPÄISCHE PATENTANMELDUNG

(11) **EP 2 383 247 A1**
(43) Veröffentlichungstag der Anmeldung: **02.11.2011**
(21) Anmeldenummer: 10004428.8
(22) Anmeldetag: 27.04.2010
(51) Int. Cl.: C07C 43/11, C11D 1/72, C11D 3/00

(54) **Verzweigte Hydroxyalkylpolyoxylenglykolether und deren Verwendung**

(71) Anmelder: Cognis IP Management GmbH, 40589 Düsseldorf (DE)
(72) Erfinder: Raths, Hans-Christian, 40789 Monheim (DE); Both, Sabine, 41470 Neuss (DE); Bauer, Frederic, 40589 Düsseldorf (DE); Weuthen, Manfred, 40764 Langenfeld (DE)

(57) **Zusammenfassung**

Bestimmte verzweigte Hydroxyalkylpolyoxylenglykolether zeigen auch bei Temperaturen von 25 °C und darunter hervorragende Eigenschaften als Entschäumer oder Schaum drückende Zusätze bzw. als Schaumregulatoren für wässerige Mittel, die Schaumbildner enthalten. Diese verzweigten Hydroxyalkylpolyoxylenglykolether können daher in Wasch- und Reinigungsmitteln eingesetzt werden.

## Beschreibung

Die vorliegende Erfindung betrifft ausgewählte verzeigte Tenside vom Typ der Hydroxyalkylpolyoxylenglykolether und deren Verwendung in Wasch- oder Reinigungsmitteln, insbesondere zur Entschäumung, Schaumkontrolle oder Schaumunterdrückung wässeriger Mittel, die schäumende Komponenten enthalten.

Wässerige Wasch- und Reinigungsmittel enthalten häufig Substanzen, die bei den Einsatzbedingungen zu einer unerwünschte Schaumbildung führen. Bei diesen Substanzen handelt es sich häufig um Anionentenside, z.B. vom Typ der Alkylbenzolsulfonate oder der linearen Alkylsulfate und Fettalkoholpolyglykolethersulfate, oder bestimmte nichtionische Tenside, wie Alkyl(oligo)glycoside, Daneben können aber auch verschiedene Bestandteile der abzureinigenden Anschmutzungen oder im Anwendungssystem befindlichen bzw. in das Anwendungssystem eingetragene Stoffe mit oberflächenaktiven Eigenschaften Schäume bilden und/oder die Schaumneigung in einer Anwendung stabilisieren und somit die Entstehung von Schäumen verursachen oder verstärken. Exemplarische sind Stoffe und Stoffgruppe wie z.B. Proteine, Polymere, verseifte Fette, Holzfasern und deren Inhaltsstoffe genannt, die Schäume bilden.
Um einer unerwünschten Schaumbildung zu Begegnen werden daher entweder a) schaumdrückende Zusätze verwendet, d.h. Substanzen, die die Entstehung von Schäumen verhindern, oder es kommen b) Entschäumer zum Einsatz, das sind Mittel, die bestehende Schäume zerstören können. Als dritte Kategorie c) sind Schaumregulatoren bekannt, das sind Substanzen, die eine Schaumbildung nicht vollständig unterbinden, aber das Schaumvolumen auf ein bestimmtes Maß begrenzen können. Details dazu finden sich in der Literatur, z.B. bei N.D. Denkov, Mechanisms of Foam Destruction by Oil-based Antifoams, Langmuir 2004, 20, 9463-9505, oder in der TEGEWA Publikation "Schaum" der Arbeitsgruppe "Oberflächenaktive Substanzen" aus dem Jahr 2007. Schäume werden durch den festen und homogenen Tensidfilm auf der Flüssigkeit stabilisiert - Es ist daher die Aufgabe eines Entschäumers, diesen Film zu zerstören, bzw. eines schaumdrückenden Zusatzes dessen Bildung zu verhindern.

Als schaumdrückende oder entschäumende Zusätze werden üblicherweise hydrophobe Öle (z.B. Mineralöl, Paraffinöle, Fettalkohole und deren Ester, Fettsäuren und Silikonöle) und deren Kombination mit fein verteilten hydrophoben Feststoffen eingesetzt. Aber auch bestimmte nichtionische Tenside können entschäumende bzw. Schaum unterdrückende Wirkung zeigen. Hier werden zum Beispiel Hydroxyalkylpolyoxylenglykolether eingesetzt. Es handelt sich dabei um bekannte Verbindungen, die z.B. durch Umsetzung eines 1,2-Epoxyalkans mit einem alkoxylierte Fettalkohol erhalten werden können. Hier wird als Beispiel für den Stand der Technik die DE 19738866 A1 herangezogen. In der EP 0 299 360 A2 z.B. werden solche Verbindungen, und deren Verwendung als schaumdrückende Zusätze offenbart. Obwohl die breite Markush-Formel der EP 0 299 360 A2 auch verzweigte Derivate umfasst, werden in der Schrift dem Fachmann aber nur Verbindungen mit linearen Alkylresten konkret offenbart. In der EP 1 229 103 A1 werden Hydmxypolyoxylenglykolether und deren Einsatz in Reinigungsmitteln offenbart, wobei in der Beschreibung auch die Verwendung verzweigter Alkohole zur Herstellung beschrieben wird, ohne dass konkrete Verbindungen dieses Typs oder deren Eigenschaften offenbart würden.

Obwohl bereits viele Substanzen bekannt sind, die als Entschäumer bzw. schaumuntordrückende Zusätze in wässerigen Mitteln geeignet sind, werden weiterhin neue Substanzen gesucht. Da aktuelle Wasch- und Reinigungsmittel bei Verbrauchern und industriellen bzw. gewerblichen Anwendern gefragt sind, die ihre Wirkung bei möglichst niedrigen Einsatztemperaturen (vorzugsweise weniger als 30 °C und insbesondere bei Temperaturen von weniger als 25 °C) entfalten, werden auch Entschäumer bzw. schaumunterdrtickende Zusätze oder Schaumregulatoren gesucht, die entweder bei diesen niedrigen Temperaturen die gleiche Wirksamkeit zeigen, wie bekannte Verbindungen bei höheren Einsatztemperaturen, oder bei gleichen Temperaturen eine bessere Wirksamkeit zeigen als Substanzen des Standes der Technik.

Es wurde nun gefunden, dass bestimmte, ausgewählte Hydroxyalkylpolyoxylenglykolether diese gewünschten Eigenschaften zeigen.

Ein erster Gegenstand der vorliegenden Erfindung betrifft daher Hydroxyalkylpolyoxylenglykolether der allgemeinen Formel (I) 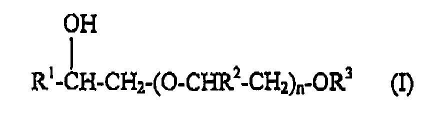 in der R¹ für einen gesättigten linearen Alkylrest mit 6 bis 18 C-Atomen steht, R² ein Wasserstoffatom oder einen Methylrest bedeutet wobei der Rest R³ für einen einfach oder mehrfach verzeigten, gesättigten Alkyrest mit 5 bis 24 C-Atomen steht und der Index n für ganze oder gebrochene Zahlen von 2 bis 20 steht.

Hydroxyalkylpolyoxylenglykolether sind im Prinzip bekannt. In der oben bereits zitierten EP 299 360 A2 finden sich in der Beschreibung auch Hinweise auf verzweigte Reste R¹ oder R³ ohne das Verbindungen gemäß der Beschreibung der vorliegenden Formel (I) konkret und unzweideutig vorbeschrieben worden wären.

Die Lehre der vorliegenden Erfindung basiert nun auf der überraschenden Erkenntnis, dass bei ausschließlicher Variation des Polyoxyglykol-terminalen Restes R³ die entschäumende bzw. schaumunterdrückende Wirkung der Hydroxyalkylpolyglykolether bei niedrigen Temperaturen erhalten bleibt, wohingegen die aus dem Stand der Technik konkret offenbarten rein linearen Derivate ihre Wirksamkeit einbüßen,

Die Verbindungen der allgemeinen Formel (I) werden üblicherweise durch Umsetzung von alkoxylierten verzweigten Fettalkoholen mit Alkyloxiranen in Gegenwart alkalischer Katalysatoren hergestellt. Die Herstellverfahren sind an sich bekannt und werden auch in der EP 0 299 364 A2 offenbart. Die verzweigten Reste R³ in der allgemeinen Formel (I) stammen also aus dem Alkohol, der vor der Umsetzung mit dem Oxiran alkoxyliert worden ist.

Als verzweigte Alkohole kommen z.B. die vier Isopentanole, insbesondere der Isoamylalkohol (3-Methylbutan-lol) in Frage, sowie als weitere Beispiele 2-Methylpentyl-, 2-Methylhexyl-, 2-Methylheptyl-, 2-Methyloctyl-, 2-Methylnonyl-, 2-Methyldecylalkohol, sowie 2-ethylpropyl-, 2-Ethylbutyl-, 2-Ethylpentyl-, 2-Ethylhexyl-, 2-Ethylheptyl-, 2-Ethyloctyl-, 2-Ethylnonyl-, 2-Ethyldecylalkohol in Frage. Auch 3-Methyl- oder 3-Ethylpentanol, -hexanol, -heptanol, -octanol, und - nonanol sind geeignete verzweigte Alkohole. Auch Isononanole oder Isodecanole sind geeignete verzweigte Alkohole im Sinne der vorliegenden Lehre. Neben den einfach verzweigten Alkoholen können auch mehrfach verzweigte Alkohole mit 6 bis 12 C-Atomen geeignet sein. Die Alkohole können auch in Form beliebiger Mischungen untereinander eingesetzt werden. Es können vorzugsweise auch alle durch Guerbet-Reaktion erhaltenden verzweigten Alkohole mit 6 bis 12 C-Atomen bzw. deren Mischungen besonders geeignet sein.
Besonders bevorzugt ist der 2-Ethylhexyalkohol oder das Isooctanol. 2-Ethylhexanol ist ein synthetischer Alkohol, der auf Propen als Rohstoff basiert. Propen wird mit CO und Wasserstoff zum Butanal umgesetzt, welches dann durch Aldolkondensation zu 2-Ethyl-3-hydroxyethanol umgesetzt wird. Nach Wasserabspaltung und katalytischer Hydrierung erhält man 2-Ethylhexanol.
Isooctanol kann auch durch sogenannte Oxo-Synthese aus Olefinen, Kohlenmonoxid (CO) und Wasserstoff erhalten werden. Diese Synthese nennt man auch Hydroformylierung. Die bei der Hydroformylierung gebildeten Aldehyde werden anschließend hydriert. Je nach eingesetztem Olefin werden verschiedene primäre Isoalkohole erhalten. So erhält man aus einem Isohepten-Gemisch durch Hydroformylienmg und anschließender Hydrierung eine Mischung, bestehend zumeist aus 3,3-, 3,5- und 4,5-Dimethyl-1-hexanol neben 3- und 5-Methyl-1-heptanol. Auch 6-Methyl-1-heptanol ist ein verbreiteter großtechnisch verfügbarer Isooctanol. Weitere besonders geeignete verzweigte Alkohole sind das Isononanol und Isodecanol, auch hier liegen Gemische vor. Isononanol ist nämlich ein Gemisch verzweigter primärer Isononanole mit einem großen Anteil von 3,5,5-Trimethyl-hexan-1-ol. Beim Isodecanol liegt ein Gemisch von isomeren Trimethylheptanolen neben 3,5-Dimethyloctan-1-ol vor.

Die Hydroxyalkylpolyoxylenglykolether der allgemeinen Formel (I) können Ethylenoxidgruppen, Propylenoxidgruppen oder sowohl Ethylenoxid- als auch Propylenoxidgruppen zusammen enthalten, wobei diese dann entweder blockweise oder randomisiert im Molekül vorliegen können. Sofern Propylenoxid-Gruppen enthalten sind folgen diese der allgemeinen Formel (Ia):

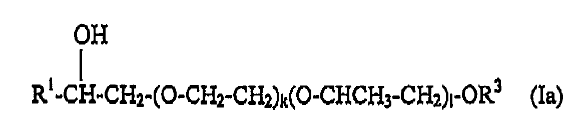

Wobei die Reste R¹ und R³ die oben genannte Bedeutung haben und der Index k eine ganze oder gebrochene Zahl von 1 bis 16 bedeutet, und 1 für eine ganze oder gebrochene Zahl von 1 bis 4, vorzugsweise 1 bis 3 und insbesondere 1 bis 2 steht, mit der Maßgabe, dass k + 1= n ist. Der Wert der Index k in der allgemeinen Formel (Ia) liegt dann bei 2 bis 16, vorzugsweise 4 bis 12 und insbesondere 6 bis 10.
Bevorzugte Verbindungen dieses Typs enthalten z.B. 2 mol Propylenoxid und 10 mol Ethylenoxid pro mol des Hydroxyalkylpolyoxylenglykolethers, oder 14 mol Ethylenoxid und 3 mol Propylenoxid pro Mol des Hydroxyalkylpolyoxylenglykolethers.

Die Reihenfolge der Gruppen (O-CH₂-CH₂)ₖ(O-CHCH₃-CH₂)ₗ in der Formel (Ia) legt keine Reihenfolge fest, vielmehr umfasst bzw. steht diese Formel für alle denkbaren blockweise wie randomisiert verteilte Gruppen (O-CH2-CH₂)ₖ und (O-CHCH₃-CH₂)ₗ innerhalb der Markush-Formel (Ia). Es werden dabei insbesondere solche Verbildungen der Formel (Ia) ausgewählt, bei denen der Anteil an Propylenoxid-Gruppen (O-CHCH₃-CH₂) kleiner ist, als der Anteil an Ethylenoxidgruppen (O-CH₂-CH₂). Es ist auch möglich z.B. rein ethoxylierte und rein propoxylierte Verbindungen der Formel (I) nebeneinander zu verwenden.
Besonders bevorzugte Verbindungen der allgemeinen Formel (I) enthalten aber immer Ethylenoxid, vorzugsweise enthalten sie nur Ethylenoxidgruppen, sind also frei von Propylenoxid-Gruppen.

Der Alkoxylierungsgrad, in der allgemeinen Formel (I) (d.h. der Anteil an Alkoxidgruppen, also Ethylenoxid und/oder Propylenoxidgruppen) durch den Index n charakterisiert, beträgt vorzugsweise von 3 bis 20, bzw. von 5 bis 15 und insbesondere von bis 6 bis 11.

In einer besonders bevorzugten Ausführungsform werden solche Moleküle der Formel (I) ausgewählt, bei denen R² für ein Wasserstoffatom steht (es handelt sich hier um ausschließlich ethoxylierte Derivate), und der Rest R³ für einen 2-Ethylhexyl-Rest steht. Ebenfalls bevorzugt sind Hydroxyalkylpolyoxylenglykolether nach der allgemeinen Formel (I) in der R³ für einen 2-Ethylhexylrest steht und n eine ganzzahlige oder gebrochene Zahl von 6 bis 10 und insbesondere von 7 bis 9 bedeutet und R¹ einen linearen Alkylrest mit 6 bis 12 C-Atomen bedeutet.

Der Rest R¹ stammt aus dem Oxiran welches bei der Umsetzung zum Einsatz kommt. Vorzugsweise werden für R¹ in der Formel (I) lineare gesättigte Alkylreste mit 6 bis 12, vorzugsweise 6 bis 10 und insbesondere 8 bis 10 C-Atomen ausgewählt.

Die Verbindungen nach der allgemeinen Formel (I) eignen sich zum Entschäumen wie auch als schaumdrückende Zusätze oder als Schaumregulatoren für wässerige Systeme, Mittel oder Lösungen, Emulsionen oder Dispersionen, die schäumenden und/oder schaumstabilisierende Komponenten enthalten. Neben schäumenden oberflächenaktiven Substanzen, wie Tensiden, können auch andere Substanzen, die in erster Linie aus Anschmutzungen eingetragen werden, wie Proteine, Polymere, verseifte Fette, Holzfasern und deren Inhaltsstoffe sowie Saponine Schäume bilden und/oder stabilisieren und somit als schäumende Komponenten, Schaumstabilisator und/oder Schaumbildner wirken.

Die Verbindungen der allgemeinen Formel (I) können auch als Schaumregulatoren eingesetzt werden. Als schäumende Tenside kommen insbesondere anionische Tenside, hier vorzugsweise Alkylsulfate, Alkylethersulfate, Alkysulfonate und/oder Alkylbenzolsulfonate in Frage. Als weitere Schaumbildner wären schäumende nichtionische Tenside wie beispielsweise Alkyl(oligo)glycoside und alkoxylierte, vorzugsweise ethoxylierte Fettalkohole, aber auch Aminoxide, Betaine, Fettsäureamide und deren alkoxylierten Derivate und andere kationische Tenside zu nennen.

Unter Alkyl- und/oder Alkenylsulfaten, die auch häufig als Fettalkoholsulfate bezeichnet werden, sind die Sulfatierungsprodukte primärer Alkohole zu verstehen, die der Formel (II) folgen,

R⁴O-SO₃X (II)

in der R⁴ für einen linearen oder verzweigten, aliphatischen Alkyl- und/oder Alkenylrest mit 6 bis 22, vorzugsweise 12 bis 18 Kohlenstoffatomen und X für ein Alkali- und/oder Erdalkalimetall, Ammonium, Alkylammonium, Alkanolammonium oder Glucammonium steht. Typische Beispiele für Alkylsulfate, die im Sinne der Erfindung Anwendung finden können, sind die Sulfatierungsprodukte von Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen, die durch Hochdruckhydrierung technischer Methylesterfraktionen oder Aldehyden aus der Roelen'schen Oxosynthese erhalten werden. Die Suliatierungsprodukte können vorzugsweise in Form ihrer Alkalisalze und insbesondere ihrer Natriumsalze eingesetzt werden. Besonders bevorzugt sind Alkylsulfate auf Basis von C_{16/18}-Talgfettalkoholen bzw. pflanzliche Fettalkohole vergleichbarer C-Kettenverteilung in Form ihrer Natriumsalze.

Alkylethersulfate ("Ethersulfate") stellen bekannte anionische Tenside dar, die großtechnisch durch SO₃- oder Chlorsulfonsäure (CSA)-Sulfatierung von Fettalkohol- oder Oxoalkoholpolyglycolethem und nachfolgende Neutralisation hergestellt werden. Im Sinne der Erfindung kommen Ethersulfate in Betracht, die der Formel (III) folgen,

R⁵O-(CH₂CH₂O)ₘSO₃X (III)

in der R⁵ für einen linearen oder verzweigten Alkyl- und/oder Alkenylrest mit 6 bis 22 Kohlenstoffatomen, n für Zahlen von 1 bis 10 und X für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 mol und insbesondere 2 bis 5 mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 2 bis 3 mol Ethylenoxid an technische C_{12/14}- bzw. C_{12/18}- Kokosfottalkoholfraktionen in Form ihrer Natrium- und/oder Magnesiumsalze.

Alkylbenzolsulfonate folgen vorzugsweise der Formel R-Ph-SO₃X in der R für einen verzweigten, vorzugsweise jedoch linearen Alkylrest mit 10 bis 18 Kohlenstoffatomen, Ph für einen Phenylrest und X für X für ein Alkali- und/oder Erdalkalimetall, Ammonium, Alkylammonium, Alkanolammonium oder Glucammonium steht. Vorzugsweise werden Cumolsulfonat, xylolsulfonat, Toluolsulfonat, Dodecylbenzolsulfonate, Tetradecylbenzolsulfonate. Hexadecylbentolsulfonate sowie deren technische Gemische z.B. in Form der Natriumsalze eingesetzt.

Alky(oligo)glycoside ebenfalls bekannt. Alkyl- und Alkenyloligoglykoside stellen bekannte nichtionische Tenside dar, die der Formel (IV)

R⁶O-[G]ₚ (IV)

folgen in der R⁶ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner/gleich 1,7 ist und insbesondere zwischen 1,2 und 1,6 vorzugsweise 1,2 bis 1,4 liegt. Der Alkyl- bzw. Alkenylrest R⁶ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP =1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁶ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylallcohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Die Wirksamkeit der Verbindungen der allgemeinen Formel (I) bei der Entschäumung oder als Schaumdrücker bzw. als schaumkontrollierender Zusatz ist bei wässerigen Systemen, Emulsionen oder Lösungen, die Alkyl(oligo)glycoside enthalten besonders ausgeprägt und somit die Verwendung hierbei bevorzugt.

Pottalkoholethoxylate folgen der allgemeinen Formel (V)

R⁷-(OC₂H₄)_{z}OH (V)

in der R⁷ für lineare oder verzweigte Alkyl- und/oder Alkenylreste mit 8 bis 22 Kohlenstoffatomen steht und z eine Zahl von 1 bis 30 und vorzugsweise von 1 bis 15, und insbesondere von 1 bis 10 bzw. bis 5 steht. Typische Beispiele sind die Addukte von durchschnittlich 1 bis 20 mol an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind Addukte von 10 bis 40 mol Ethylenoxid an technische Fettalkohole mit 12 bis 18 Kohlenstoffatomen, wie beispielsweise Kokos-, Palm", Palmkern- oder vorzugsweise Talgfettalkohol. Besonders bevorzugte Fettalkoholethoxylate basieren auf Talgalkohole, die mit 5 bis 25 und vorzugsweise 7 bis 15 mol Ethylenoxid pro mol Alkohol ethoxyliert sind.

Die Verbindungen der allgemeinen Formel (I) zeichnen sich dabei darin aus, dass sie ihre schaumunterdrückenden bzw. entschäumende oder schaumkontrollierende Wirkung bei niedrigeren Temperaturen als z.B. die linearen Isomeren oder andere Mittel des Standes der Technik wie Silikonentschäumer zeigen. Die Wirkung tritt bereits bei Temperaturen von weniger als 30 °C, insbesondere Temperaturen von kleiner/gleich 25 °C und sogar bei 20 °C und niedrigeren Temperaturen auf. Außerdem können die Verbindungen der allgemeinen Formel (I) mit einer Vielzahl anderer Tenside und sonstigen Inhaltsstoffen von Wasch- und Reinigungsmitteln kombiniert und formuliert werden. Die erfindungsgemäßen Mittel haben aber nicht, wie man es bei herkömmlichen entschäumdenen Zusätze, wie z.B. hydrophobe Öle (z.B. Mineralöl, Paraffinöle, Fettalkohole und deren Ester, Fettsäuren und Silikonöle) beobachtet, einen nachteiligen Einfluss oder Effekt auf sonstige Reinigungskomponenten oder auf das Reinigungsergebnis oder die Reinigungsleistung. Durch den Zusatz der Verbindungen der allgemeinen Formel (I) ist sogar unerwarteter Weise eine Verbesserung der Reinigungsleistung in Reinigungsanwendungen zu beobachten.

Die vorliegende Lehre umfasst auch ein Verfahren zum Entschäumen von wässerigen Tensidsystemen (das umfasst auch wässerige Emulsionen oder dispersionen) vorzugsweise von Lösungen, wobei man bei Temperaturen von kleiner gleich 25 °C Verbindungen gemäß der allgemeinen Formel (I) in Mengen von mindestens 25 ppm, vorzugsweise von mindesten 50 und insbesondere von mindestens 100 ppm (mg/kg Tensidlösung) der wässerigen Tensidlösung zusetzt. Die Zugabe kann auch portionsweise erfolgen. Es ist auch möglich, eine wässerige Lösung, enthaltend die Verbindungen der Formel (I) vorzulegen, und diese dann mit dem zu entschäumenden Medium in Kontakt zu bringen bzw. dieses zuzusetzen.
Die Verbindungen der allgemeinen Formel (I) können somit vorteilhaft als Additiv verwendet werden, um z.B. in bestehenden oder neu zu entwickelnden Formulierungen von Wasch- und Reinigungsmittel zum Entschäumen, als Schaumdrücker oder als Schaumregulator Verwendung zu finden.

Die Verbindungen der Formel (I) zeigen weiterhin besondere vorteilhafte Eigenschaften: So tritt bei Silikon-Entschaumern - in der Regel handelt es sich hier um mit Silikonölen hydropho-modifizierte Silica-Partikel - keine dauerhafte Wirkung auf, d.h., dass man immer wieder Entschäumer zudosieren muss, um neue Schaumbildung zu vermeiden. Die Verwendung der erfindungsgemäßen Hydroxyalkylpolyoxylenglykolether gemäß der allgemeinen Formel (I) führt bereits durch einmalige Zugabe zu einer dauerhaften entschäumenden Wirkung. Damit wird die Menge an Entschäumer-Substanzen deutlich verringert, da die Menge an Aktivsubstanz gegenüber den Silikonentschäumern nicht erhöht werden muss. Außerdem wirken die Substanzen gemäß der vorliegenden Lehre im Vergleich zu anderen flüssigen Entschäumern schneller, eine Eigenschaft, die bei vielen Anwendungen vorteilhaft ist.
Außerdem sind die Verbindungen der allgemeinen Formel (I) in der Lage auch Schäume, die durch Alkyl(oligo)glycoside gebildet werden wirksam zu unterdrücken bzw. zu vermeiden. Eine Eigenschaft, die andere Entschäumer nicht ausweisen.

Die Verbindungen der allgemeinen Formel (I) können dabei zur Entschäumung bzw. als schaumdrückende Zusätze oder als Schaumregulatoren für alle Arten wässeriger Lösungen, aber auch für alle Arten von Emulsionen (insbesondere vom Typ W/O und O/W) oder Dispersionen eingesetzt werden. Es kann sich z.B. um Waschflotten oder Reinigungsnüttelösungen handeln, bei denen keine oder nur eine geringe Schaumentwicklung auftreten soll.

Besonders bevorzugt können die Hydroxypolyoxylenglykolether der allgemeinen Formel (I) für Reinigungsmittel im Bereich der industriellen und institutionellen Reinigung harter Oberflächen, z.B. bei der Reinigung von Flaschen aus Glas, Kunststoff oder Metall eingesetzt werden. Auch verschiedensten Sprtireinigungsanwendungen oder so genannten CIP-Reinigunsanwendungen (cleaning in place) die entschäumende, schaumunterdrtlokende oder schaumkontrollierende Zusätze in der Anwendung erfordern wie es z.B. bei der Reinigung von Tanks (z.B. zur Lagerung von flüssigen Lebensmitteln, die Schaumbildner enthalten, wie bei der Bierherstellung) können derartige Reinigungsmittel mit Vorteil verwendet werden. Ein weiteres Anwendungsgebiet sind schaumkontrollierte Produkte für die Oberflächenpflege von Fußbodenbelägen mit manuellen, halbautomatisierten oder automatisierten Reinigungsgeräten.
Durch die Reduzierung der Temperatur der wässerigen Tensidlösung kann z.B. der Energieeinsatz bei der Reinigung gegenüber herkömmlichen Entschäumern verringert werden, oder man kann die Einsatzmenge bei gleicher Temperatur verringern.

Ein weiterer Gegenstand der vorliegenden Erfindung betrifft Wasch- Spül-, und Reinigungsmittel, die Verbindungen der allgemeinen Formel (I) enthalten, vorzugsweise in Mengen von 0,001 bis 10 Gew.%, bezogen auf das Gesamtgewicht der Mittel, und insbesondere von 0,01 bis 2 Gew.-%. Diese Wasch- und Reinigungsmittel können fest, flüssig oder gelförmig sein und sie enthalten neben den Verbindungen der allgemeinen Formel (I) noch weitere charakteristische Inhaltsstoffe, wie Tenside, Bleichmittel und -aktivatoren, Buildersubstanzen, Komplexbildner, Hydrotope, nicht-wässerige Lösungsmittel, Polymere, Farb- und Duftstoffe, anorganische und/oder organische pH-Regulantien, Konservierungsmittel, Biozide und/oder Verdicker.

### Beispiele

### Herstellung der Hydroxypolyoxylenglykolether:

2-Ethylhexylalkohol bzw. n-Octanol wurden jeweils in Gegenwart von Natriummethylat als Katalysator mit Ethylenoxid bei 160 °C zum ethoxylierten Alkohol mit 9 mol Ethylenoxid pro mol Alkohol umgesetzt und anschließend neutralisiert. Der ethoxylierte Alkohol aus dem ersten Schritt wurde anschließend in einem zweiten Reaktionsschritt bei 160 -180 °C in Gegenwart von Natriummethylat als Katalysator mit einem C₁₀-Alkylepoxid zum gewünschten 2-Ethylhexyl-9-EO-hydroxydecylether (E1) bzw. Isooctanol-9EO-hydroxydecylether (V1) umgesetzt und neutralisiert.

### Anwendungstechnische Untersuchungen;

Die Verbindungen E1 und V1 wurden jeweils auf ihre Eignung als schaumkontrollierende und schaumdrückende Mittel bzw. als Entschäumer untersucht:

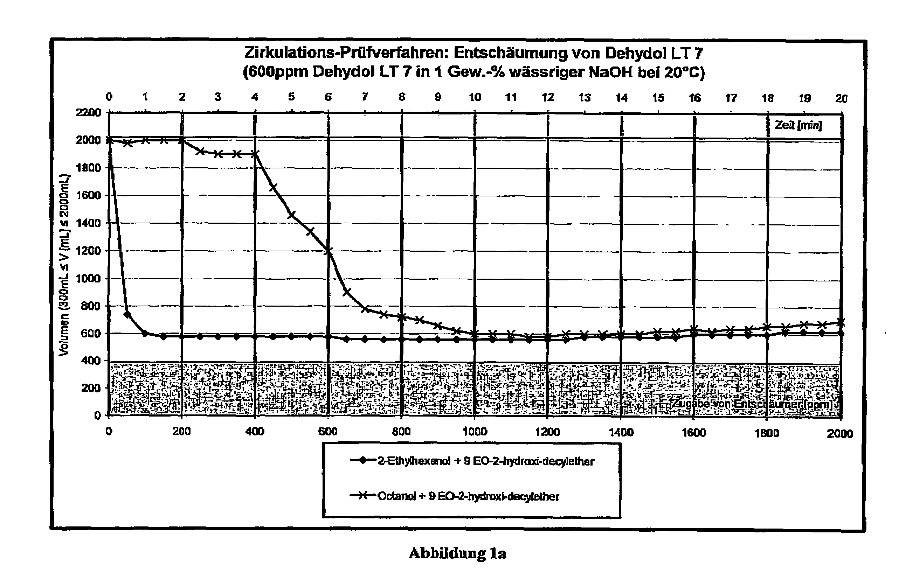

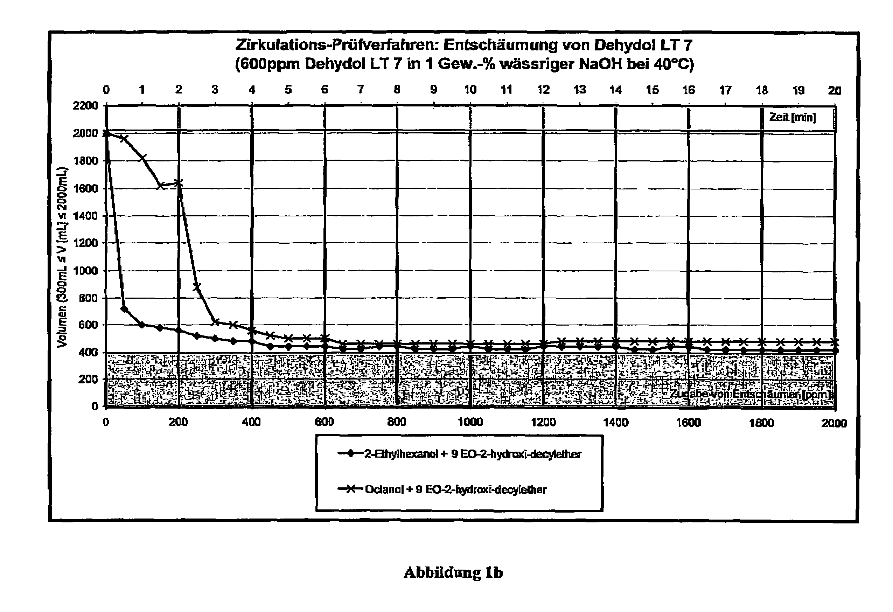

Die Messung der entschäumenden Wirkung erfolgte gemäß der DIN Methode 14371:2004 in einem Zirkulationsprüfverfahren.
Es wurden bei 20 °C und bei 40 °C 500 mL einer 1 Gew.-% wässerigen NaOH-Lösung mit 600 ppm eines schäumenden nichtionischen Tensids vom Typ der Fettalkoholethoxylate (Dehydol® LT 7 der Fa. Cognis) versetzt und ein Schaum mit einem Aktivschaumvolumen von 2000 mL erzeugt. Zu diesem Schaum wurden dann unterschiedliche Mengen der Verbindungen E1 bzw. V1 zur Entschäumung zugesetzt. Die Ergebnisse sind in der Abbildung 1a (20 °C) und Abbildung 1b (40 °C) wiedergegeben.
Es zeigt sich, dass die erfindungsgemäße Verbindung E1 jeweils bereits bei einer niedrigeren Dosierung eine entschäumende Wirkung im Vergleich zur Substanz V1 zeigt.

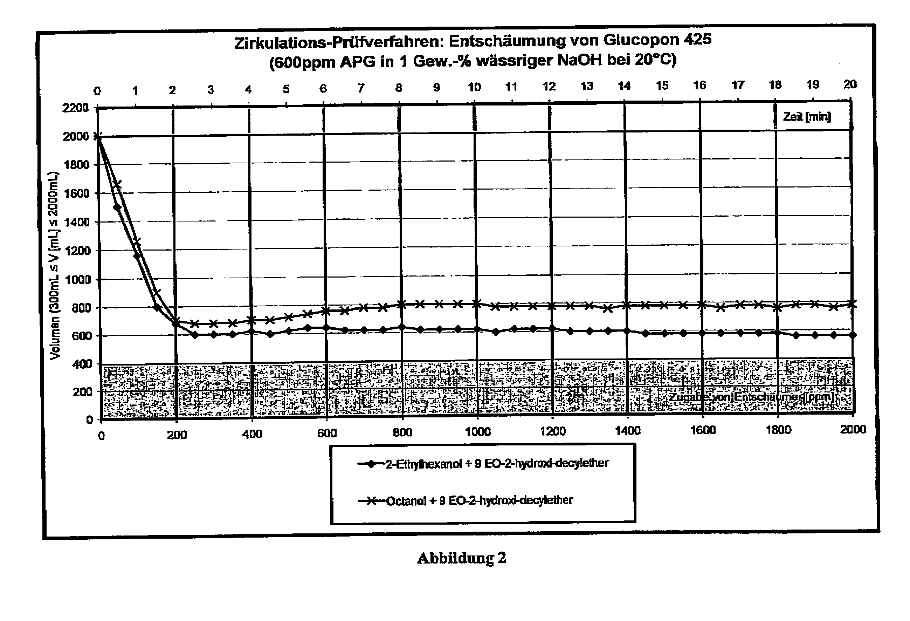

In einem weiteren Test wurde wie oben verfahren, allerdings wurde statt des Dehydol ^{®}LT 7 ein Alkyl(oligo)glycosid (Glucopon^{®} 425 N/HH der Fa. Cognis) als Tensid verwendet. Wieder wurden die Substanzen E1 und V1 zugesetzt und die entschäumende Wirkung in Abhängigkeit von der zudosierten Menge gemessen. Die Ergebnisse finden sich in der Abbildung 2. Auch hier wirkt das erfindungsgemäße Mittel E1 besser als die Vergleichssubstanz V1.

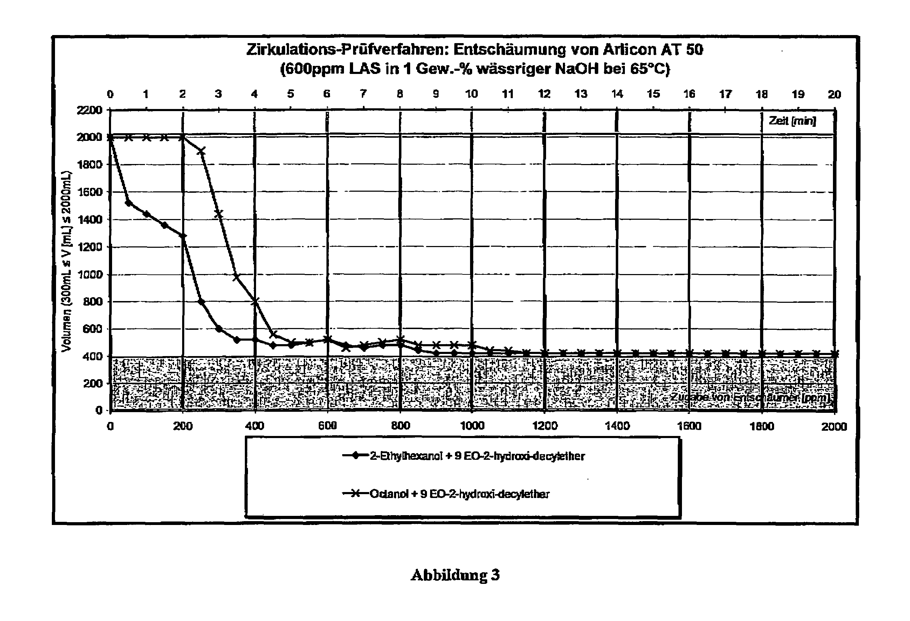

In einem weiteren Test, der analog zu den obigen Versuchen durchgeführt wurde, wurde als schäumendes Tensid ein anionisches Tensid vom Typ der Alkylbenzolsulfonate (Arlicon^{®} AT 50) verwendet. Die Messtemperatur betrug diesmal 65 °C. Die Ergebnisse der Messung finden sich in der Abbildung 3. Wieder zeigt das erfindungsgemäße verzweigte Produkt E1 eine bessere Wirksamkeit als das lineare Vergleichsprodukt V1.

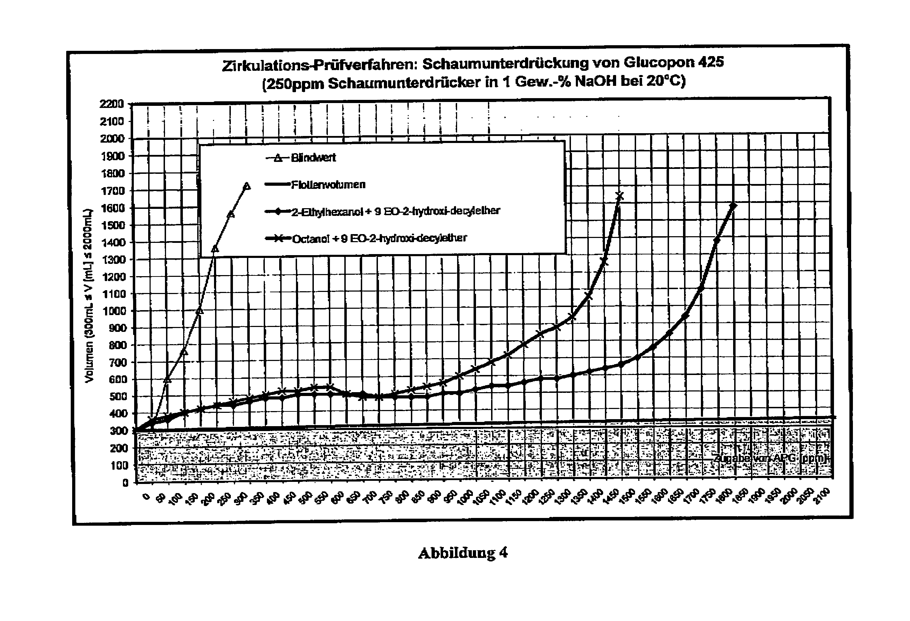

Zur Messung der schaumunterdrückenden Wirkung wurde mittels eines Zirkulationsprüfverfahrens bei 20 °C zu 400 mL einer wässerigen NaOH-Lösung (1 Gew.-%) einmal 250 ppm E1 und in einem zweiten Versuch 250 ppm V1 zugegeben. Anschließend wurde alle 30 Sekunden jeweils in Schritten von 50 ppm das stark schäumende Alkyl(oligo)glycosid Cilucopon^{®} 425 N/HH zudosiert und die Schaumentwicklung gemessen. Die Ergebnisse finden sich in der Abbildung 4. E1 zeigt eine bessere Schaumunterdrückung als V1.

Es wurde weiterhin das Reinigungsvermögen der erfindungsgemäßen Verbindung E1 gegenüber anderen entschäumenden Tenside des Standes der Technik gemessen. Dazu wurden jeweils 1 Gew.-% des Tensids (Aktivsubstanzgehalt) in destilliertem Wasser bei 25 °C in einem Gardner-Test auf PVC mit einer Standardanschmutzung (IPP 83/21) geprüft. Zum Vergleich wurden neben E1 ein propoxylierte Fettalkohol (V2) und eine Silikonemulsion (V3) eingesetzt. E1 erreichte eine Reinigungsleistung von 75 %, V2 38 % und V3 45 %.

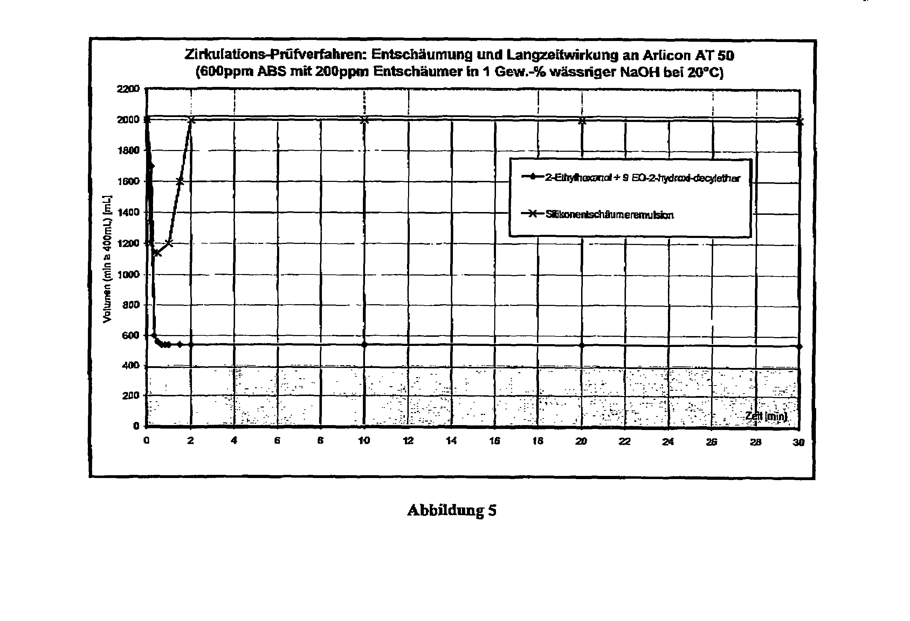

In einem weiteren Test wurden die Verbindung E1 mit einem handelsüblichen Silikonentschäumer (TEGO^{®} Antifoam 1488 - Fa. Evonik) verglichen. Als Schaumbildner kam das Arlicon^{®} AT 50 zum Einsatz. Wie aus Abbildung 5 zu entnehmen ist, entschäumt E1 wirksamer - außerdem bleibt die Wirkung auch über einen langen Zeitraum erhalten, wohingegen der Silikonentschäumer keine nachhaltige schaumkontrollierende Wirkung entfalten kann.

## Patentansprüche

1. Hydroxyalkylpolyoxylenglykolether der allgemeinen Formel (I) 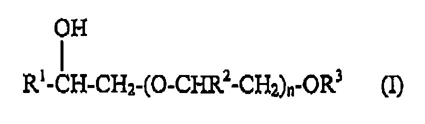 in der R¹ für einen gesättigten linearen Alkylrest mit 6 bis 18 C-Atomen steht, R² ein Wasserstoffatom oder einen Methylrest bedeutet **dadurch gekennzeichnet, dass** R³ für einen einfach oder mehrfach verzeigten, gesättigten Alkyrest mit 5 bis 24 C-Atomen bedeutet und n für ganze oder gebrochene Zahlen von 2 bis 20 steht.

2. Hydroxyalkylpolyoxylenglykolether nach Anspruch 1, **dadurch gekennzeichnet, dass** R² für ein Wasserstoffatom und der Rest R³ für einen 2-Bthylhexyl-Rest oder einen Isooctanol-Rest steht.

3. Hydroxyalkylpolyoxylenglykolether nach mindestens einem der Ansprüche 1 bis 2, **dadurch gekennzeichnet, dass** R¹ für einen gesättigten linearen Alkylrest mit 6 bis 12, vorzugsweise 6 bis 10 und insbesondere 8 bis 10 C-Atomen steht.

4. Hydroxyalkylpolyoxylenglykolether nach mindestens einem der Ansprüche 1 bis 3, **dadurch gekennzeichnet, dass** der Index n in der allgemeinen Formel (I) für gerade oder gebrochene Zahlen von 3 bis 20, vorzugsweise von 5 bis 15 und insbesondere von 6 bis 11 steht.

5. Hydroxyalkylpolyoxylenglykolether nach mindestens einem der Ansprüche 1 bis 4, **dadurch gekennzeichnet, dass** in der allgemeinen Formel (I) R³ für einen 2-Ethylhexylrest steht und n eine geradzahlige oder gebrochen Zahl von 6 bis 10 und insbesondere von 7 bis 9 bedeutet und R¹ einen linearen Alkylrest mit 6 bis 12 C-Atomen bedeutet.

6. Hydroxyalkylpolyoxylenglykolether nach mindestens einem der Ansprüche 1 bis 5, **dadurch gekennzeichnet, dass** R² in der allgemeinen Formel (I) für ein Wasserstoffatom steht.

7. Verwendung von Hydroxyalkylpolyoxylenglykolether der allgemeinen Formel (I) gemäß der Beschreibung im Anspruch 1 als Entschäumer oder schaumkontrollierender oder schaumdrückender Zusatz für wässerige Mittel, die Schaumbildner enthalten.

8. Verwendung nach dem Anspruch 7, **dadurch gekennzeichnet, dass** die entschäurne,n,de schaumkontrollierende oder schaumdrückende Wirkung bereits bei Temperaturen von kleiner/gleich 25 °C, vorzugsweise von kleiner/gleich 20 °C auftritt.

9. Wasch-, Spül- und Reinigungsmittel, enthaltend Hydroxyalkylpolyoxylenglykolether der allgemeinen Formel (I) gemäß der Beschreibung im Anspruch 1.

10. Verfahren zum Entschäumen von wässerigen Mitteln, enthaltend Schaumbildner, **dadurch gekennzeichnet, dass** man bei Temperaturen von kleiner/gleich 25°C, vorzugsweise kleiner/gleich 20 °C Verbindungen gemäß der allgemeinen Formel (I) wie im Anspruch 1 beschrieben, der wässerigen Mittel in Mengen von mindestens 25 ppm zusetzt.
